# EUROPEAN PATENT SPECIFICATION

(11) **EP 3 838 226 B1**
(45) Date of publication and mention of the grant of the patent: **17.08.2022**
(21) Application number: 19217202.1
(22) Date of filing: 17.12.2019
(51) Int. Cl.: A61F 2/30, A61F 2/38

(54) **PROSTHETIC COMPONENT**
PROTHESENKOMPONENTE
COMPOSANT PROTHÉTIQUE

(43) Date of publication of application: 23.06.2021
(73) Proprietor: implantcast GmbH, 21614 Buxtehude (DE)
(72) Inventor: ROLBIECKI, Patryk, 21641 Apensen (DE); SAß, Jens, 21614 Buxtehude (DE)
(74) Representative: Uexküll & Stolberg

(56) References cited:
- EP-A1- 2 710 980
- US-A- 5 152 797
- US-B2- 6 896 702
- US-B2- 9 918 845

## Description

### TECHNICAL FIELD

The present invention relates to the field of knee prostheses. In particular the present invention relates to a prosthetic component adapted to be mounted to a bone as part of a knee prosthesis.

### BACKGROUND OF THE INVENTION

Orthopedic prosthesis are commonly utilized to repair and/or replace damaged bone and tissue in the human body. In a total knee arthroplasty procedure, anterior, distal and posterior portions of the natural femur are resected. A femoral component is then installed on the femur in place of the resected bone. The tibia is also resected and a tibial component is installed on the tibia in place of the resected bone, accordingly. Both the femoral component and the tibial component are fixed to the corresponding bone via bone cement. An inlay is located on the tibial component and fixed thereto. The femoral component is designed to articulate with the inlay during flexion and extension of the knee.

Ten to fifteen years after a first total knee arthroplasty the prosthetic components are worn and have to be replaced in a so called knee revision. During a knee revision the femoral component and the tibial component are mechanically separated from the bone, i.e. the femur and the tibia. Subsequently, the bone surfaces are cleaned and prepared for new prosthetic components to be installed thereon. Due to the mechanical separation of the prosthetic components defects in the bone may occur. In order to correct these defects and to provide a stable supporting surface for the prosthetic components additional bone may be resected. In order to compensate the resected bone spacing elements are used which are fixed to a contact surface of the femoral and/or tibial component. These spacing elements are available in different sizes and thicknesses and are for example made from metal.

With regard to tibial components the spacing elements are in general fixed to the tibial components via a fixation mechanism, in particular via a screw connection. Depending on the construction the screws are either screwed through the spacing element into a thread in the tibial component or through the tibial component into a thread in the spacing element.

When installing a screw through the tibial component into the spacing element, the problem of backside wear arises. Micro movements between an inlay receiving surface of the tibial component to which the inlay is fixed and a corresponding attachment surface of the inlay may lead to abrasion of the attachment surface of the inlay. This effect is amplified the rougher the receiving surface of the tibial component is manufactured. As a head of a screw, the screw extending through the tibial component into the spacing element, is never fully plain with the receiving surface of the tibial component, backside wear of the inlay is increased.

When installing a screw through the spacing element into the tibial component this disadvantage is overcome. In this case the inlay receiving surface of the tibial component does not comprise a hole. Accordingly, backside ware of the inlay is decreased. However, in order to provide a stable screw connection, sufficient convolutions of the thread and thus a sufficient depth of the core hole of the thread in the tibial component have to be provided. This requires a main body of the tibial component which is thicker than a main body used when installing a screw through the tibial component into the spacing element. This increased thickness contravenes the main aim when constructing a tibial component which is to provide a tibial component with a main body having a thickness as low as possible in order to install as less external material to the body as possible.

Several manufacturers of tibial components solve this problem by providing two different tibial components, namely a primary tibial component for a first total knee arthroplasty and a secondary tibial component for a knee revision. The primary tibial component does not have any possibility to fix spacing elements thereto and can thus be manufactured with a thin main body. The secondary tibial component comprises a much thicker main body and comprises one or more threads with sufficient convolutions.

The same situation arises with regard to femoral components.

US9918845 proposes an augment attachment mechanism via a screw inserted into a slotted nut and thereby expanding the nut's cut-out bottom portion to lock the augment to the prosthetic component.

A similar mechanism is proposed by EP2710980.

Furthermore, there is US5152797 disclosing a rotatable eccentric insert which, when rotated, locks an augment to the prosthetic component. However, the mechanism of EP2710980 does not comprise a fastening element.

It is therefore an object of the present invention to provide one single prosthetic component which can be used for a primary total knee arthroplasty as well as for a knee revision.

### SUMMARY OF THE INVENTION

This object is achieved by a prosthetic component comprising the features of claim 1. Preferred embodiments are set out in the dependent claims.

According to the present invention the fixation mechanism comprises a receiving portion located at the contact surface, an insert for insertion into the receiving portion and a fastening element adapted to engage with the insert. The receiving portion is adapted to fix the insert via a bayonet joint connection such that the fastening element, extending through the spacing element and engaging with the insert, fixes the spacing element to the contact surface.

Due to the use of an insert a thinner main body of the prosthetic component compared to a conventional main body with a thread installed therein can be used. Preferably, the receiving portion does not extend through the main body of the tibial component to a surface of the main body opposing the contact surface. Consequently, roughness of the surface opposing the contact surface and thus backside wear on said surface is preferably minimized. A bayonet joint connection between the receiving portion and the insert allows an easy installation of the insert and guarantees a durable connection at the same time. Preferably, the insert is inserted into the receiving portion and subsequently the bayonet joint connection is established. The spacing element is installed on the contact surface thereafter and the fastening element is brought into engagement with the insert through the spacing element.

According to an embodiment of the present invention the insert has a cylindrical shape and comprises multiple protrusions which extend radially outward. Preferably, the multiple protrusions are located at a lower end of the insert.

In another embodiment of the present invention the protrusions extend in opposing directions. Preferably the insert comprises two protrusions. The cylindrical shape together with the radially extending protrusions may form an oval base of the insert.

In another embodiment of the present invention the receiving portion comprises a non-cylindrical receiving opening which allows insertion of the insert into the receiving portion in a first position of the insert. Preferably, the non-cylindrical opening corresponds to the shape of the lower end of the insert, in particular to the oval base of the insert formed by the cylindrical shape together with the opposing radially extending protrusions. Optionally, the receiving opening of the receiving portion is located at the contact surface.

Preferably, the receiving portion comprises undercuts which are configured to prevent removal of the insert from the receiving portion in a second position of the insert. Preferably, the undercuts are located below the contact surface of the main body of the prosthetic component and point towards a bottom of the receiving portion. Preferably, the undercuts are formed by overhangs of the main body protruding into the receiving portion.

In an embodiment of the present invention the receiving portion is configured such that upon movement of the insert from the first position into the second position the protrusions of the insert are brought into engagement with the undercuts of the receiving portion and the bayonet joint connection between the receiving portion and the insert is established. In connection with the present invention the term "the bayonet joint connection is established" is understood as a condition in which removal of the insert out of the receiving portion along an axis extending normal to the contact surface is prevented. Preferably, the insert is inserted into the receiving portion and then rotated from the first position into the second position in order to bring the opposing protrusions of the insert into engagement with the undercuts of the receiving portion. Preferably, the receiving portion is configured to allow lifting of the insert due to engagement of the fastening element and the insert from a bottom of the receiving portion towards the undercuts.

In another embodiment of the present invention the second position is offset from the first position by an angle of between 80 degrees and 100 degrees, preferably of 90 degrees around a axis of rotation which extends normal to the contact surface. Rotation of the insert from the first to the second position allows establishing the bayonet joint connection in an easy way.

Preferably, the receiving portion is formed as a blind hole. The surface opposing the contact surface of the prosthetic component does not have a trough hole and roughness of the opposing surface is thus kept to a minimum.

In and embodiment of the present invention the insert is a threaded insert in the fastening element is a screw. A screw connection is an easy, efficient and cost efficient way to fix the fastening element to the insert. A screw connection guarantees proper fixation of the spacing element to the contact surface when being implanted into a human body.

In another embodiment of the present invention the prosthetic component is a tibial component and the bone to which the prosthetic component is adapted to be mounted is a tibia. Preferably the tibial component is formed of a cobalt chrome alloy and/or stainless steel.

Preferably, the tibial component has an inlay receiving surface located opposite the contact surface, wherein the inlay receiving surface is adapted to abut an inlay which is configured to articulate with a femoral component in a knee prosthesis. The inlay receiving surface may form the surface as mentioned above opposing the contact surface of the main body of the prosthetic component. Due to the use of an insert the inlay receiving surface is plain and roughness of the inlay receiving surface is set to a minimum in order to avoid backside wear of the inlay.

In an embodiment of the present invention the prosthetic component is a femoral component and the bone to which the prosthetic component is adapted to be mounted is a femur. The fixation mechanism may also be used in combination with a femoral component. A bearing surface of the femoral component which is formed by condyles of the femoral component does thus not comprise a screw hole in order to fix the spacing plate through the femoral component to the contact surface of the femoral component. Roughness of the bearing surface is thus minimized. Preferably, the bearing surface forms the surface as mentioned above opposing the contact surfaces of the main body of the prosthetic component.

The above object is also achieved by a knee prosthesis comprising the features of claim 12. According to the present invention the knee prosthesis comprises a prosthetic component as mentioned above.

The invention will now be described in connection in one exemplary embodiment shown in the Figures in which:
- Figure 1: shows a knee prosthesis in an isometric view,
- Figure 2: shows a perspective view of a tibial component with an insert,
- Figure 3: shows a sectional view of the insert of Figure 2 inserted into a receiving portion,
- Figure 4: shows a perspective view of the tibial component of Figure 2 together with a spacing element,
- Figure 5: shows a sectional view of the tibial component of Figure 4 and a fixation mechanism and
- Figure 6: shows a sectional view of a tibial component of Figure 5 together with a spacing element being fixed to the tibial component.

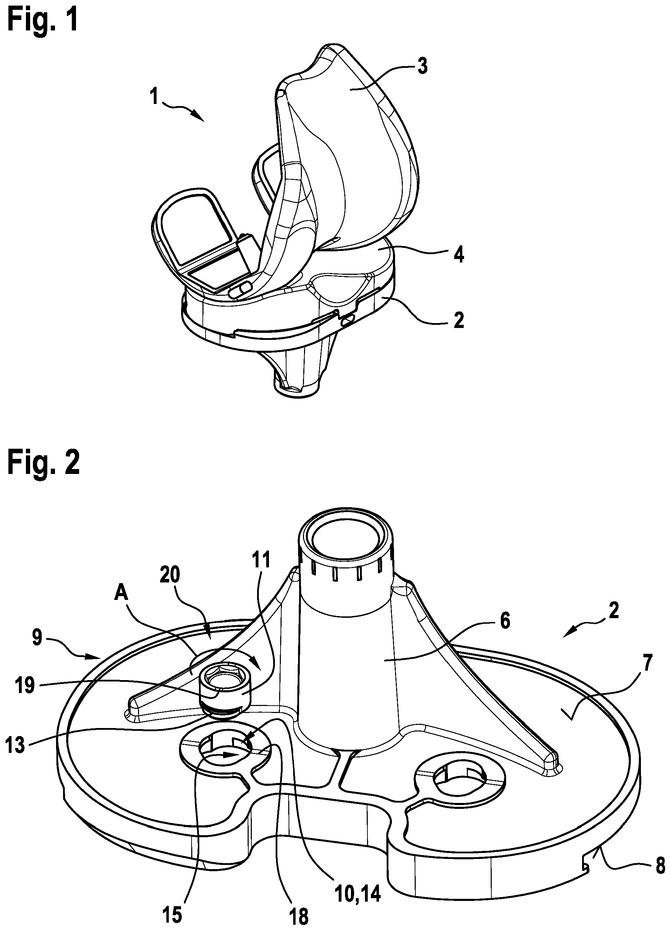

Figure 1 shows a knee prosthesis 1 with a first prosthetic component formed as a tibial component 2, a second prosthetic component formed as a femoral component 3 and an inlay 4. The tibial component 2 and the femoral component 3 are adapted to be mounted onto a tibia (not shown) and a femur (not shown), respectively. The inlay 4 is located between the femoral component 3 and the tibial component 2 and is fixed to the latter. The femoral component 3 is adapted to articulate with the inlay 4 to allow a flexion of the knee prosthesis 1.

Figures 2 to 4 show the tibial component 2 in detail. The tibial component 2 comprises a spacing element 5 for compensating resected bone, in particular tibial bone. The tibial component 2 further comprises a main body 6 with a contact surface 7 configured to abut the spacing element 5 and an inlay receiving surface 8 located opposite the contact surface 7. The inlay receiving surface 8 is adapted to abut the inlay 4.

The tibial component 2 further comprises a fixation mechanism 9 for fixing the spacing element 5 to the main body 6. The fixation mechanism 9 includes a receiving portion 10 located at the contact surface 7, a threaded insert 11 for insertion into the receiving portion 10 and a fastening element formed as a screw 12. The screw 12 is adapted to be inserted into a throughole 21 extending through the spacing element 5 and to engage with the threaded insert 11. The insert 11 has a cylindrical shape and comprises two protrusions 13 extending radially outward in opposing directions at a lower end 23 of the insert 11. The protrusions 13 form an oval base 24 of the insert 11.

The receiving portion 10 is formed as a blind hole 14 which has a receiving opening 15 at the contact surface 7. The opening 15 is non-cylindrical, in particular oval, and allows insertion of the insert into the receiving portion 10 in a first position of the insert 11 (as shown in Figure 2). The shape of the opening 15 corresponds to the shape of the oval base 24 of the insert 11.

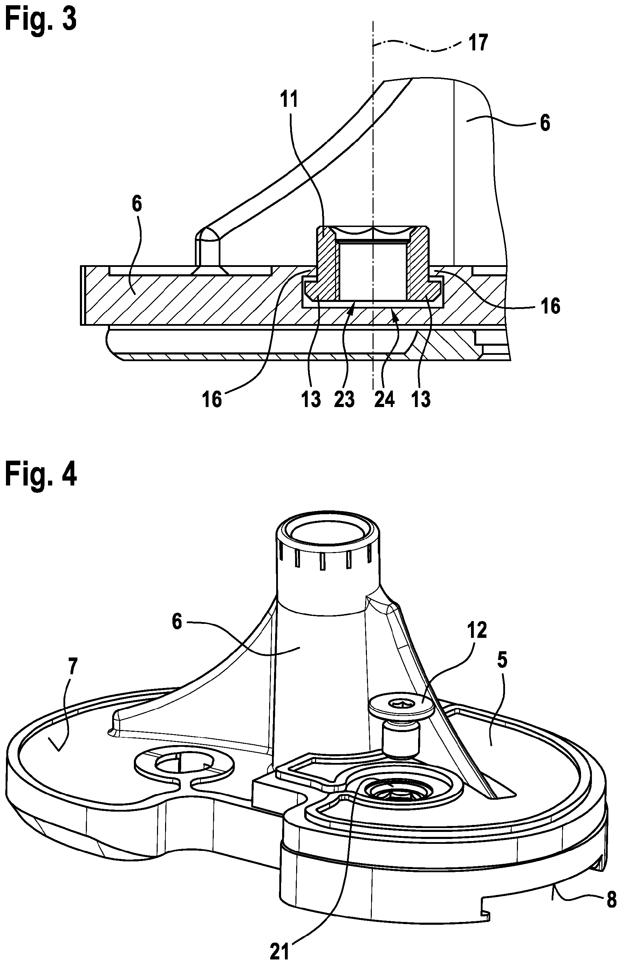

The receiving portion 10 comprises undercuts 16, which are configured to prevent removal of the insert 11 from the receiving portion 10 in a second position of the insert 11 (as shown in Figure 3) in a direction normal to the contact surface 7. The second position is off-set from the first position by an angel of 90 degrees around an axis of rotation 17 which extends normal to the contact surface 7. In order to move the insert 11 from the first position to the second position the insert 11 has to be turned clockwise along arrow A around the axis of rotation 17.

The contact surface 7 comprises opposing first markings 18 located in proximity to the receiving portion 10. The insert 11 comprises corresponding second markings 19 at its top end 20. The first and second markings 18, 19 are configured such that upon alignment of the first markings 18 with the second markings 19 the insert is in its second position (as shown in Figure 3).

The receiving portion 10 is further configured such that upon movement of the insert 11 from the first position (as shown in Figure 2) into the second position (as shown in Figure 3) the protrusions 13 of the insert 11 are brought into engagement with the undercuts 16 of the receiving portion 10 and the bayonet joint connection between the receiving portion 10 and the insert 11 is established. Removal of the insert 11 from the receiving portion 10 in a direction normal to the contact surface 7 is prevented.

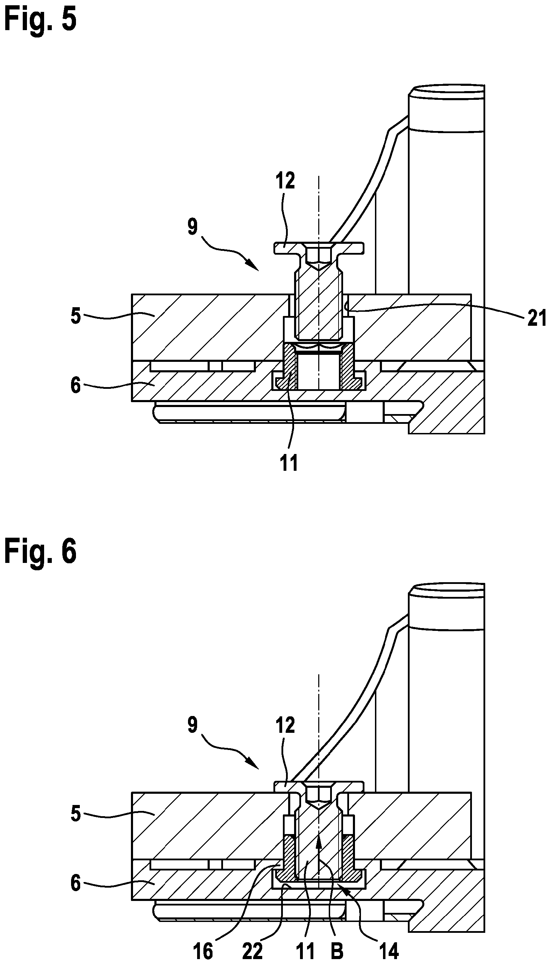

As can be seen in Figures 4 to 6 the screw 12 extends through the throughhole 21 of the spacing element 5 and engages with the threaded insert 11. The receiving portion 10 is further configured such that upon tightening of the screw 12 against the spacing element 5 the insert 11 is lifted along arrow B from a bottom 22 of the blind hole 14 towards the undercuts 16 of the receiving portion until the protrusions 13 of the insert 11 abut the undercuts 16 of the receiving portion 10. In other words, the receiving portion 10 is adapted to fix the insert 11 via a bayonet joint connection such that the screw 12, extending through the spacing element 5 and engaging with the insert 11, fixes the spacing element 5 to the contact surface 7.

### Reference numerals

- 1: knee prosthesis
- 2: tibial component (first prosthetic component)
- 3: femoral component (second prosthetic component)
- 4: inlay
- 5: spacing element
- 6: main body
- 7: contact surface
- 8: inlay receiving surface
- 9: fixation mechanism
- 10: receiving portion
- 11: insert
- 12: fastening element
- 13: protrusions
- 14: blind hole
- 15: receiving opening
- 16: undercuts
- 17: axis of rotation
- A: arrow
- 18: first markings
- 19: second markings
- 20: top end
- 21: through hole
- 22: bottom B arrow
- 23: lower end (insert)
- 24: oval base (insert)

## Claims

1. Prosthetic component adapted to be mounted to a bone as part of a knee prosthesis (1), the prosthetic component (2, 3) comprising:
a spacing element (5) for compensating resected bone,
a main body (6) with a contact surface (7) configured to abut the spacing element (5) and
a fixation mechanism (9) for fixing the spacing element (5) between the contact surface (7) and the bone,
the fixation mechanism (9) comprises:
a receiving portion (10) located at the contact surface (7),
an insert (11) for insertion into the receiving portion (10) and
a fastening element (12) adapted to engage with the insert (11),
**characterized in that**
the receiving portion (10) is adapted to fix the insert (11) via a bayonet joint connection such that the fastening element (12), extending through the spacing element (5) and engaging with the insert (11), fixes the spacing element (5) to the contact surface (7).

2. Prosthetic component according to claim 1, **characterized in that** the insert (11) has a cylindrical shape and comprises multiple protrusions (13) which extend radially outward.

3. Prosthetic component according to claim 1, **characterized in that** the protrusions (13) extend in opposing directions.

4. Prosthetic component according to any of the preceding claims, **characterized in that** the receiving portion (10) comprises a non-cylindrical receiving opening (15) which allows insertion of the insert (11) into the receiving portion (10) in a first position of the insert (11).

5. Prosthetic component according to any of the preceding claims, **characterized in that** the receiving portion (10) comprises undercuts (16) which are configured to prevent removal of the insert (11) from the receiving portion (10) in a second position of the insert (11).

6. Prosthetic component according to claims 2, 4 and 5, **characterized in that** the receiving portion (10) is configured such that upon movement of the insert (11) from the first position into the second position the protrusions (13) of the insert (11) are brought into engagement with the undercuts (16) of the receiving portion (10) and the bayonet joint connection between the receiving portion (10) and the insert (11) is established.

7. Prosthetic component according to claim 6, **characterized in that** the second position is offset from the first position by an angle of between 80° and 100°, preferably of 90° around an axis of rotation (17) which extends normal to the contact surface (7).

8. Prosthetic component according to any of the preceding claims, **characterized in that** the receiving portion (10) is formed as a blind hole (14).

9. Prosthetic component according to any of the preceding claims, **characterized in that** that the insert is a threaded insert (11) and the fastening element is a screw (12).

10. Prosthetic component according to any of the preceding claims, **characterized in that** the prosthetic component is a tibial component (2) and the bone to which the prosthetic component is adapted to be mounted is a tibia.

11. Prosthetic component according to claim 10, **characterized in that** the tibial component (2) has an inlay receiving surface (8) located opposite the contact surface (7), wherein the inlay receiving surface (7) is adapted to abut an inlay (4) which is configured to articulate with a femoral component (3) in a knee prosthesis (1).

12. Prosthetic component according to any of claims 1 to 9, **characterized in that** the prosthetic component is a femoral component (3) and the bone to which the prosthetic component is adapted to be mounted is a femur.

13. Knee prosthesis comprising a prosthetic component (2, 3) according to any of claims 1 to 12.

## Patentansprüche

1. Prothetikkomponente, die geeignet ist, an einem Knochen als Teil einer Knieprothese (1) befestigt zu werden, wobei die Prothetikkomponente (2, 3) umfasst:
ein Abstandselement (5) zum Ausgleich von reseziertem Knochen,
einen Hauptkörper (6) mit einer Kontaktfläche (7), die ausgebildet ist, an dem Abstandselement (5) anzuliegen, und
einen Fixierungsmechanismus (9) zum Fixieren des Abstandselements (5) zwischen der Kontaktfläche (7) und dem Knochen,
der Fixierungsmechanismus (9) umfasst:
einen Aufnahmeabschnitt (10), der sich an der Kontaktfläche (7) befindet,
einen Einsatz (11) zum Einsetzen in den Aufnahmeabschnitt (10) und
ein Befestigungselement (12), das ausgebildet ist, mit dem Einsatz (11) in Eingriff gebracht zu werden,
**dadurch gekennzeichnet, dass**
der Aufnahmeabschnitt (10) geeignet ist, den Einsatz (11) über eine Bajonettverbindung zu fixieren, so dass das Befestigungselement (12), das sich durch das Abstandselement (5) erstreckt und mit dem Einsatz (11) in Eingriff steht, das Abstandselement (5) an der Kontaktfläche (7) fixiert.

2. Prothetikkomponente nach Anspruch 1, **dadurch gekennzeichnet, dass** der Einsatz (11) eine zylindrische Form hat und mehrere Vorsprünge (13) aufweist, die sich radial nach außen erstrecken.

3. Prothetikkomponente nach Anspruch 1, **dadurch gekennzeichnet, dass** sich die Vorsprünge (13) in entgegengesetzte Richtungen erstrecken.

4. Prothetikkomponente nach einem der vorhergehenden Ansprüche, **dadurch gekennzeichnet, dass** der Aufnahmeabschnitt (10) eine nicht zylindrische Aufnahmeöffnung (15) aufweist, die das Einsetzen des Einsatzes (11) in den Aufnahmeabschnitt (10) in einer ersten Position des Einsatzes (11) ermöglicht.

5. Prothetikkomponente nach einem der vorhergehenden Ansprüche, **dadurch gekennzeichnet, dass** der Aufnahmeabschnitt (10) Hinterschneidungen (16) aufweist, die ausgebildet sind, das Entfernen des Einsatzes (11) aus dem Aufnahmeabschnitt (10) in einer zweiten Position des Einsatzes (11) zu verhindern.

6. Prothetikkomponente nach einem der Ansprüche 2, 4 und 5, **dadurch gekennzeichnet, dass** der Aufnahmeabschnitt (10) so ausgebildet ist, dass bei einer Bewegung des Einsatzes (11) von der ersten Position in die zweite Position die Vorsprünge (13) des Einsatzes (11) mit den Hinterschneidungen (16) des Aufnahmeabschnitts (10) in Eingriff gebracht werden und die Bajonettverbindung zwischen dem Aufnahmeabschnitt (10) und dem Einsatz (11) hergestellt wird.

7. Prothetikkomponente nach Anspruch 6, **dadurch gekennzeichnet, dass** die zweite Position gegenüber der ersten Position um einen Winkel zwischen 80° und 100°, vorzugsweise um 90°, um eine senkrecht zur Kontaktfläche (7) verlaufende Drehachse (17) versetzt ist.

8. Prothetikkomponente nach einem der vorhergehenden Ansprüche, **dadurch gekennzeichnet, dass** der Aufnahmeabschnitt (10) als Sackloch (14) ausgebildet ist.

9. Prothetikkomponente nach einem der vorhergehenden Ansprüche, **dadurch gekennzeichnet, dass** der Einsatz ein Gewindeeinsatz (11) und das Befestigungselement eine Schraube (12) ist.

10. Prothetikkomponente nach einem der vorhergehenden Ansprüche, **dadurch gekennzeichnet, dass** die Prothetikkomponente eine Tibiakomponente (2) ist und der Knochen, an dem die Prothetikkomponente befestigbar ist, eine Tibia ist.

11. Prothetikkomponente nach Anspruch 10, **dadurch gekennzeichnet, dass** die Tibiakomponente (2) eine Einlagenaufnahmefläche (8) aufweist, die sich gegenüber der Kontaktfläche (7) befindet, wobei die Einlagenaufnahmefläche (7) geeignet ist, an einer Einlage (4) anzuliegen, die ausgebildet ist, mit einer Femurkomponente (3) in einer Knieprothese (1) ein Gelenk zu bilden.

12. Prothetikkomponente nach einem der Ansprüche 1 bis 9, **dadurch gekennzeichnet, dass** die Prothetikkomponente eine Femurkomponente (3) ist und der Knochen, an dem die Prothetikkomponente befestigbar ist, ein Femur ist.

13. Knieprothese mit einer Prothetikkomponente (2, 3) nach einem der Ansprüche 1 bis 12.

## Revendications

1. Composant prothétique adapté pour être monté sur un os en tant que partie d'une prothèse de genou (1), le composant prothétique (2, 3) comprenant :
un élément d'espacement (5) pour compenser l'os réséqué,
un corps principal (6) ayant une surface de contact (7) conçue pour porter contre l'élément d'espacement (5) et
un mécanisme de fixation (9) pour fixer l'élément d'espacement (5) entre la surface de contact (7) et l'os,
le mécanisme de fixation (9) comprenant :
une partie de réception (10) située au niveau de la surface de contact (7),
un insert (11) destiné à être inséré dans la partie de réception (10) et
un élément de fixation (12) adapté pour venir en prise avec l'insert (11),
**caractérisé en ce que**
la partie de réception (10) est adaptée pour fixer l'insert (11) via une connexion à baïonnette de sorte que l'élément de fixation (12), qui s'étend à travers l'élément d'espacement (5) et vient en prise avec l'insert (11), fixe l'élément d'espacement (5) à la surface de contact (7).

2. Composant prothétique selon la revendication 1, **caractérisé en ce que** l'insert (11) a une forme cylindrique et comprend de multiples saillies (13) qui s'étendent radialement vers l'extérieur.

3. Composant prothétique selon la revendication 1, **caractérisé en ce que** les saillies (13) s'étendent dans des directions opposées.

4. Composant prothétique selon l'une quelconque des précédentes revendications, **caractérisé en ce que** la partie de réception (10) comprend une ouverture de réception non cylindrique (15) qui permet l'insertion de l'insert (11) dans la partie de réception (10) dans une première position de l'insert (11).

5. Composant prothétique selon l'une quelconque des précédentes revendications, **caractérisé en ce que** la partie de réception (10) comprend des contre-dépouilles (16) qui sont conçues pour empêcher le retrait de l'insert (11) de la partie de réception (10) dans une seconde position de l'insert (11).

6. Composant prothétique selon les revendications 2, 4 et 5, **caractérisé en ce que** la partie de réception (10) est conçue de sorte que, lors du mouvement de l'insert (11) de la première position dans la seconde position, les saillies (13) de l'insert (11) sont amenées en prise avec les contre-dépouilles (16) de la partie de réception (10) et la connexion à baïonnette entre la partie de réception (10) et l'insert (11) est établie.

7. Composant prothétique selon la revendication 6, **caractérisé en ce que** la seconde position est décalée par rapport à la première position d'un angle allant de 80° à 100°, de préférence de 90° autour d'un axe de rotation (17) qui s'étend perpendiculairement à la surface de contact (7).

8. Composant prothétique selon l'une quelconque des précédentes revendications, **caractérisé en ce que** la partie de réception (10) est formée comme un trou borgne (14).

9. Composant prothétique selon l'une quelconque des précédentes revendications, **caractérisé en ce que** l'insert est un insert fileté (11) et l'élément de fixation est une vis (12).

10. Composant prothétique selon l'une quelconque des précédentes revendications, **caractérisé en ce que** le composant prothétique est un composant tibial (2) et l'os sur lequel le composant prothétique est destiné à être monté est un tibia.

11. Composant prothétique selon la revendication 10, **caractérisé en ce que** le composant tibial (2) a une surface de réception d'inlay (8) située en face de la surface de contact (7), où la surface de réception d'inlay (8) est adaptée pour porter contre un inlay (4) qui est conçu pour s'articuler avec un composant fémoral (3) dans une prothèse de genou (1).

12. Composant prothétique selon l'une quelconque des revendications 1 à 9, **caractérisé en ce que** le composant prothétique est un composant fémoral (3) et l'os sur lequel le composant prothétique est destiné à être monté est un fémur.

13. Prothèse de genou comprenant un composant prothétique (2, 3) selon l'une quelconque des revendications 1 à 12.
